Europäisches Patentamt

European Patent Office

Office européen des brevets

(19)

(11) Numéro de publication : **0 076 714**
**B1**

(12) FASCICULE DE BREVET EUROPÉEN

(45) Date de publication du fascicule du brevet :
02.01.86

(21) Numéro de dépôt : **82401662.0**

(22) Date de dépôt : **13.09.82**

(51) Int. Cl.⁴ : **A 61 F   2/30**

(54) Prothèse articulaire améliorée en polymère et son procédé de préparation.

(30) Priorité : **24.09.81 FR 8118013**

(43) Date de publication de la demande :
**13.04.83 Bulletin 83/15**

(45) Mention de la délivrance du brevet :
**02.01.86 Bulletin 86/01**

(84) Etats contractants désignés :
**BE CH DE GB IT LI LU NL SE**

(56) Documents cités :
**FR-A- 2 184 159**
**FR-A- 2 440 733**
**US-A- 3 839 743**

(73) Titulaire : **COMMISSARIAT A L'ENERGIE ATOMIQUE
Etablissement de Caractère Scientifique Technique
et Industriel
B.P. 510
F-75752 Paris Cedex 15 (FR)**

(72) Inventeur : **Gaussens, Gilbert
11, rue Jean Brunet
F-92190 Meudon (FR)**
Inventeur : **Nicaise, Maryvonne
7, allée des Bathes
F-91400 Orsay (FR)**
Inventeur : **Tran, Kieu Oanh
24 rue des Chardonnerets
F-91940 Les Ulis (FR)**

(74) Mandataire : **Mongrédien, André et al
c/o BREVATOME 25, rue de Ponthieu
F-75008 Paris (FR)**

# 0 076 714

## Description

La présente invention a pour objet une prothèse articulaire améliorée en polymère et son procédé de préparation.

De façon plus précise, elle concerne une prothèse articulaire partielle ou totale dont les propriétés ont été améliorées, d'une part, en ce qui concerne les coefficients de frottement de sa surface de glissement et, d'autre part, en ce qui concerne sa résistance à la déformation.

Depuis quelques années les problèmes posés par le traitement de maladies telles que l'arthrose de la hanche peuvent être résolus par des interventions chirurgicales qui consistent à supprimer les parties atteintes de l'articulation et à les remplacer par des prothèses métalliques, céramiques ou plastiques afin de retrouver les coefficients de frottement compatibles avec un fonctionnement convenable de l'articulation.

En effet, l'articulation humaine a un coefficient de frottement particulièrement faible et les propriétés spécifiques du liquide synovial et du cartilage rendent l'articulation apte à répondre aux besoins qu'exigent un travail de frottement sous contrainte et de longue durée.

Lorsqu'il est nécessaire de recourir aux prothèses articulaires, on n'envisage pas de remplacer l'ensemble des mécanismes qui contrôlent une articulation saine, mais seulement de mettre en place de nouvelles surfaces articulaires tout en favorisant l'adaptation à la prothèse du contrôle neuromusculaire, du système ligamentaire, de la capsule et de la synoviale dont on essaie de préserver les fonctions.

Pour faciliter cette adaptation, on réalise les prothèses de façon à reproduire le plus fidèlement possible la géométrie et les formes naturelles de l'articulation. Par ailleurs, le choix des matériaux utilisés pour la prothèse est très important compte tenu des propriétés de glissement qu'elle doit présenter et des forces qui s'exercent sur les points charnière que sont les articulations. Ainsi, les surfaces articulaires de glissement doivent présenter un frottement aisé mais ne peuvent s'user exagérément car il est nécessaire d'éviter la formation de débris d'usure qui, selon leur granulométrie, pourraient provoquer des réactions inflammatoires.

Jusqu'à présent, on a réalisé les prothèses articulaires en métal, en céramique ou en matière plastique.

Les prothèses métalliques présentent l'inconvénient de conduire à la formation de débris d'usure très mal tolérés lorsque les surfaces de glissement de l'articulation sont toutes deux réalisées en métal. Aussi, on les a pratiquement abandonnées en raison de ces défauts.

Les prothèses en céramique présentent l'avantage de posséder de très faibles coefficients de frottement lorsqu'elles sont utilisées soit en contact direct avec la partie saine d'une articulation, soit en contact avec une partie en polyéthylène ou en céramique. Cependant elles présentent l'inconvénient d'être fragiles et cassantes, ce qui entraîne des accidents de rupture in vitro.

Les prothèses en matière plastique sont généralement utilisées en contact avec une partie métallique, ce qui permet d'obtenir un coefficient de frottement très bas de l'articulation. Le métal est soit un alliage de chrome-cobalt, soit de l'acier inoxydable et la prothèse en matière plastique est généralement réalisée en polyéthylène de haute densité. Cependant, ces prothèses articulaires présentent l'inconvénient de conduire à la formation de débris d'usure assez bien tolérés mais inacceptables, et de subir des déformations par fluage qui sont rédhibitoires pour le bon fonctionnement de la prothèse.

La présente invention a précisément pour objet une prothèse articulaire améliorée en matière plastique polymérisée qui permet d'éviter les inconvénients précités.

A cet effet, la prothèse articulaire en polymère comprend une surface de glissement et elle se caractérise en ce que ladite surface de glissement est constituée par une couche d'environ 0,3 à 0,5 $\mu m$ d'épaisseur en ledit polymère greffé par du tétrafluoréthylène.

Grâce à la présence de cette couche de faible épaisseur de polymère greffé par du tétrafluoréthylène, on améliore le coefficient de frottement de la prothèse, tout en conservant les propriétés mécaniques intéressantes du polymère en lequel elle est réalisée.

Le procédé employé conduit à une modification locale du polyéthylène par greffage de ($C_2 F_4$) et à la formation d'un copolymère : éthylène-tétrafluoroéthylène, qui permet une amélioration des qualités de frottement et une meilleure résistance au fluage. En effet, le phénomène de la réticulation appliquée simultanément permet d'éviter le glissement irréversible des chaînes de copolymère observé dans le cas du polytétrafluoroéthylène pur qui, sous l'effet du rayonnement, se dégrade et ne se réticule pas. Ainsi, la mobilité des chaînes polymériques augmentée par la présence d'éléments fluorés, ne conduit pas au fluage mais facilite le retour du copolymère à sa structure initiale.

Copolymère : poly-(éthylène-tétrafluoroéthylène) réticulé

$(CH_2—CH)n .... (CF_2—CF_2) .... (CH—CH_2)n$
$(CH_2—CH)n .... (CF_2—CF_2)n .... (CH—CH_2)n$

Polymère : poly-(tétrafluoroéthylène) non réticulé

$.... (CF_2—CF_2) ....$
$.... (CF_2—CF_2) ....$

Généralement, l'épaisseur de la couche de polymère greffé est d'environ 0,3 à 0,5 $\mu m$.

2

Selon un mode préféré de réalisation de l'invention, la prothèse est réalisée en polymère réticulé, ce qui permet d'améliorer encore ses propriétés mécaniques. Dans ce cas, elle comprend avantageusement une partie constituée de polymère réticulé à un degré plus important que le reste de la prothèse. L'emplacement de la partie réticulée à un degré plus important, est choisi en fonction des contraintes mécaniques subies par la prothèse pour correspondre à l'endroit où les contraintes de pression sont les plus fortes.

Ainsi, on améliore la résistance au fluage de la prothèse en créant une zone de dureté plus importante à l'endroit où les contraintes sont les plus fortes. Grâce à la structure particulière de la prothèse de l'invention, c'est-à-dire à la présence de zones de polymère réticulé à des degrés différents et d'une surface de glissement réalisée en polymère greffé par du tétrafluoréthylène, on peut obtenir des duretés appropriées et, en particulier, éviter d'avoir une dureté trop forte sur la surface de glissement, ce qui conduirait à la formation de débris abrasifs entraînant une usure plus importante.

Avantageusement, pour obtenir de bons coefficients de frottement, le taux de greffage en tétrafluoréthylène de la couche d'environ 0,3 à 0,5 $\mu$m d'épaisseur constituant la surface de glissement, est de 0,3 à 2,5 mg de tétrafluoréthylène par cm$^2$ de surface.

On précise que ce taux de greffage correspond à la formule $P_m/S$ dans laquelle $P_m$ représente la quantité en poids de tétrafluoréthylène dans la prothèse et S représente la surface externe de la couche de polymère greffé constituant la surface de glissement.

Selon l'invention, le polymère constituant la prothèse peut être choisi dans le groupe comprenant les polyoléfines telles que le polyéthylène et le polypropylène, le polystyrène, les polyacrylates, le chlorure de polyvinyle, les polyamides et les polyesters.

Le choix du polymère dépend en particulier de l'articulation que la prothèse doit remplacer et il est effectué en tenant compte des propriétés mécaniques du polymère pour obtenir les caractéristiques mécaniques souhaitées de l'articulation à remplacer (genou, épaule, cheville, doigts, etc...).

De préférence, notamment dans le cas des prothèses de la hanche, le polymère est du polyéthylène.

L'invention a également pour objet un procédé de préparation d'une prothèse présentant les caractéristiques précitées. Ce procédé consiste :

a) à revêtir d'un vernis protecteur, imperméable et inerte vis-à-vis du tétrafluoréthylène, la surface externe d'une prothèse articulaire en polymère comportant une surface de glissement sauf sur la ou les zones qui constituent cette surface de glissement,

b) à soumettre la prothèse ainsi revêtue à une irradiation au moyen de rayonnements ionisants,

c) à mettre en contact la prothèse ainsi irradiée avec de la vapeur de tétrafluoréthylène pendant une durée suffisante pour obtenir le greffage du tétrafluoréthylène sur une épaisseur d'environ 0,3 à 0,5 $\mu$m du polymère constituant la surface de glissement, et

d) à éliminer le vernis protecteur.

En opérant ainsi et en réalisant l'irradiation dans des conditions appropriées, on peut obtenir, d'une part, une réticulation du polymère constituant la prothèse et, d'autre part, le greffage du tétrafluoréthylène uniquement sur la surface de la prothèse non protégée par le vernis.

Ce vernis a simplement pour fonction de protéger la prothèse, c'est-à-dire de l'imperméabiliser pour éviter tout contact avec le monomère à l'état de vapeur et d'empêcher ainsi le greffage du monomère sur les zones de la prothèse qui sont protégées.

Ce vernis doit être imperméable et inerte vis-à-vis du tétrafluoréthylène, avoir une bonne mouillabilité vis-à-vis du polymère, avoir une bonne solidité, être peu sujet aux déchirures, rester souple et avoir une résistance suffisante aux rayonnements ionisants.

Par ailleurs, il est nécessaire qu'il ait une adhérence suffisante pour protéger efficacement la prothèse et qu'il puisse être éliminé facilement en fin d'opération, de préférence par simple décollement.

Enfin, il est préférable que celui-ci conserve ses propriétés mécaniques à basse température, par exemple à − 100 °C. En effet, pour mettre en contact la prothèse revêtue avec le monomère gazeux, on effectue généralement un transfert du monomère gazeux en refroidissant le conteneur dans lequel se trouvent les éléments de prothèse à greffer.

A titre de vernis susceptibles d'être utilisés, on peut citer les vernis à base de résines vinyliques tels que le produit désigné sous le nom de Nuclétex® qui correspond à la formulation suivante :

| | |
|---|---|
| résine vinylite VYHH | 8 parties en poids |
| (87 % de chlorure de vinyle) | |
| (13 % d'acétate de vinyle) | |
| résine vinylite VYNS | 18 parties en poids |
| (90 % de chlorure de vinyle) | |
| (10 % d'acétate de vinyle) | |
| paraflex (polyester) | 8 parties en poids |
| phtalate de diotyle | 6 parties en poids |
| solvant (méthyl-éthyl-cétone) | jusqu'à dissolution. |

Ce vernis est appliqué sur la prothèse par des procédés classiques, par exemple, au moyen d'un pinceau. Après séchage de celui-ci, on soumet la prothèse ainsi protégée à une irradiation au moyen de rayonnements ionisants, ce qui permet d'assurer, d'une part, la réticulation du polymère et, d'autre part, d'engendrer les radicaux libres qui constitueront les sites actifs pour le greffage. Cette irradiation est réalisée en l'absence d'oxygène, par exemple sous vide ou sous atmosphère de gaz inerte tel que l'azote. Les rayonnements ionisants susceptibles d'être utilisés sont les rayonnements γ, les rayonnements ultraviolets, les faisceaux d'électrons.

De préférence, on réalise cette irradiation au moyen d'un faisceau d'électrons, d'une énergie de 2,5 à 3 MeV avec une dose de 10 à 15 Mrad.

Après irradiation, la prothèse est mise en contact avec de la vapeur de tétrafluoréthylène afin de greffer celui-ci sur le polymère de la ou des zones de la prothèse non protégées par le vernis. Cette mise en contact est effectuée à une température et pendant une durée choisis en fonction du taux de greffage que l'on veut obtenir.

En effet, ce taux de greffage peut être contrôlé en agissant sur l'énergie et la dose d'irradiation appliquées, sur la pression du monomère, sur la température et sur la durée de contact avec le monomère.

Pour obtenir un taux de greffage de 0,3 à 2,5 mg/cm², on réalise la mise en contact de la prothèse avec le tétrafluoréthylène pendant une durée de 50 à 70 heures, avec une pression de tétrafluoréthylène de 1,2 à 1,6 bar.

On peut régler le taux de réticulation du polymère constituant la prothèse en agissant sur l'énergie du faisceau de rayonnements ionisants, sur la dose d'irradiation et sur l'orientation de la prothèse par rapport au faisceau.

Selon une variante de mise en œuvre du procédé de l'invention, on réalise l'irradiation de la prothèse en deux étapes pour obtenir dans la première étape la réticulation du polymère constituant la prothèse et pour effectuer dans la seconde étape la pré-irradiation nécessaire pour le greffage.

Dans ce cas, après avoir appliqué le vernis sur les parties de la prothèse à protéger, on réalise de préférence la première étape au moyen de rayonnements gamma et la seconde étape au moyen d'un faisceau d'électrons.

Toutefois, on peut effectuer l'application du vernis entre les deux étapes d'irradiation. Dans ce cas, le procédé consiste :

a) à irradier une prothèse articulaire en polymère comportant une surface de glissement au moyen de rayonnements ionisants pour réticuler ledit polymère,

b) à revêtir d'un vernis protecteur imperméable et inerte vis-à-vis du tétrafluoréthylène la surface externe de la prothèse articulaire en polymère réticulé à l'exception de la ou des zones qui constituent la surface de glissement,

c) à irradier la prothèse ainsi protégée au moyen de rayonnements ionisants,

d) à mettre en contact la prothèse ainsi irradiée avec de la vapeur de tétrafluoréthylène pendant une durée suffisante pour obtenir le greffage du tétrafluoréthylène sur une épaisseur d'environ 0,3 à 0,5 μm du polymère constituant la surface de glissement et,

e) à éliminer le vernis protecteur.

De même que précédemment, on réalise de préférence l'étape de réticulation par irradiation au moyen de rayonnements gamma et l'étape d'irradiation de la prothèse protégée par le vernis au moyen d'un faisceau d'électrons.

Pour obtenir dans la prothèse une partie constituée de polymère réticulé ayant un degré de réticulation plus important que le reste de ladite prothèse, on peut utiliser des procédés classiques, par exemple choisir l'énergie des rayonnements pour obtenir des réticulations différentes. De préférence, pour obtenir ce résultat, on réalise l'irradiation de façon telle que la prothèse reçoive le rayonnement ionisant sous deux orientations différentes.

Dans ce cas, on peut par exemple effectuer l'irradiation de la prothèse en deux étapes et modifier la position de la prothèse par rapport au faisceau de rayonnements entre les deux étapes de façon qu'une zone de la prothèse reçoive deux doses d'irradiation alors que le reste de la prothèse ne reçoit qu'une seule dose d'irradiation. Ainsi, on obtient un taux de réticulation plus important dans la zone ayant reçu les deux doses ; l'emplacement de cette zone est choisi de façon telle qu'il corresponde à l'axe d'orientation de l'articulation, qui supporte les plus fortes pressions.

D'autres caractéristiques et avantages de l'invention apparaîtront mieux à la lecture des exemples suivants donnés bien entendu à titre illustratif et non limitatif en référence au dessin annexé sur lequel :

la figure 1   représente le dispositif utilisé pour tester la résistance à la déformation des prothèses articulaires selon l'invention,

les figures 2 et 3   représentent la cinématique du dispositif d'essais,

la figure 4   représente les déformations observées sur le cotyle de la prothèse de l'exemple 1 et

la figure 5   représente les déformations observées sur le cotyle de la prothèse de l'exemple 2.

Exemple 1

Cet exemple se rapporte au traitement d'une prothèse de la hanche en polyéthylène dont la forme correspond à la partie de l'os iliaque comprenant le cotyle.

**0 076 714**

Tout d'abord, on applique au pinceau sur la surface externe de la prothèse sauf dans la cavité interne du cotyle un vernis constitué par le produit nommé Nuclétex®.

On soumet ensuite la prothèse ainsi revêtue à une irradiation gamma réalisée sous vide au moyen d'une source de cobalt 60 dans les conditions suivantes :

débit de dose : 0,27 Mrad/h
dose : 15 Mrad.

Ensuite, on soumet la prothèse à une nouvelle irradiation réalisée également sous vide au moyen d'un faisceau d'électrons ayant une énergie de 3 MeV, une intensité de faisceau de 400 μA avec une dose de 13 Mrad. Après irradiation, on introduit dans l'enceinte sous vide contenant la prothèse du tétrafluoréthylène sous une pression de 1,5 bar à une température de 15 °C et on laisse la prothèse en contact avec le tétrafluoréthylène pendant une durée de 50 h, ce qui permet d'obtenir le greffage du tétrafluoréthylène sur les parties non protégées de la prothèse. Après cette étape de greffage, on retire la couche de vernis protecteur en la décollant de la prothèse, puis on détermine le taux de greffage qui correspond à la formule $P_m/S$ avec $P_m = P_f - P_i$ où $P_f$ représente le poids de la prothèse après greffage, $P_i$ le poids de la prothèse avant greffage, et S la surface de la prothèse non protégée par le vernis.

Dans ces conditions, le taux de greffage obtenu est de 0,318 mg de tétrafluoréthylène par $cm^2$ de surface non protégée.

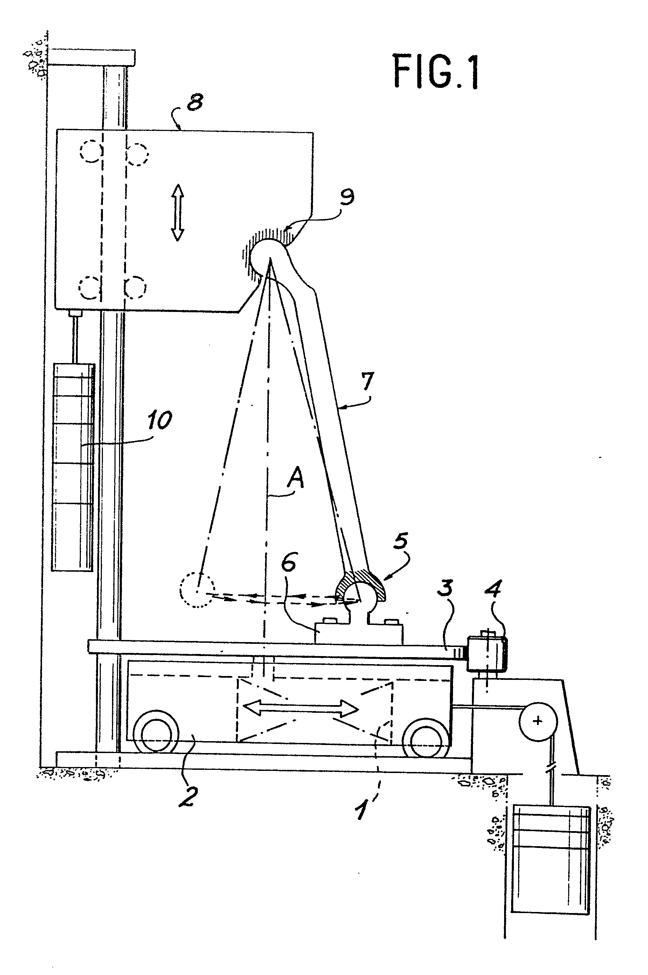

La prothèse ainsi obtenue est ensuite soumise aux tests d'usure réalisés au moyen du dispositif représenté sur la figure 1. Ce dispositif comprend un motoréducteur 1 sur lequel est fixé verticalement un chariot 2 mobile selon un axe horizontal, une came 3 solidaire du motoréducteur 1 en contact permanent avec un galet fixe 4, un ensemble manivelle 6 entraîné par le motoréducteur 1 et sur lequel est installé un cardan 5 recevant la base d'un fémur 7, un second chariot 8 perpendiculaire au premier et mobile dans un plan vertical qui supporte la prothèse 9 à tester et la charge 10. Le dispositif comprend de plus une alimentation en eau distillée qui permet d'assurer la lubrification des parties en frottement.

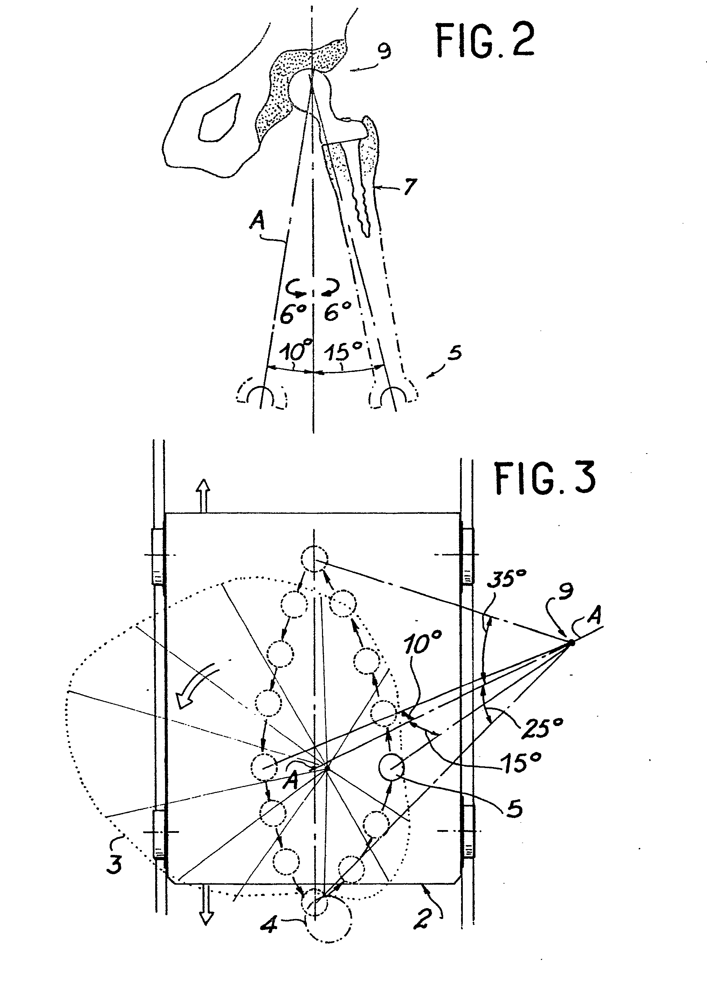

En fonctionnement, le motoréducteur 1 entraîne la manivelle 6 dont le mouvement circulaire se trouve altéré par la came 3 en contact avec le galet 4, ce qui introduit conjointement au mouvement circulaire de la manivelle 6 un déplacement alternatif du centre de rotation de la manivelle dans le plan d'action du chariot. De ce fait, le déplacement de la manivelle est modifié et prend approximativement une forme elliptique et la différence de longueur entre le grand axe et le petit axe de l'ellipse engendre au niveau de la tête fémorale deux déplacements du chariot vertical 8 supportant la prothèse 9. Une révolution de la came 3 qui assure le déplacement du fémur 7 et de la prothèse 9 correspond à un cycle. On a représenté sur la figure 2 le mouvement que décrit la tête fémorale dans le cotyle de la prothèse 9 pendant un cycle. Ce mouvement se décompose de la façon suivante.

On obtient un déplacement angulaire du fémur 7 de l'arrière vers l'avant en décrivant par rapport à la verticale suivant le sens de la marche, en premier lieu, un angle de 25° et ensuite un angle de 35°, la limite entre ces deux angles étant le plan vertical perpendiculaire à la marche dans lequel passe l'axe de symétrie A du squelette. Conjointement à ce déplacement latéral vers l'extérieur, on a un déplacement d'une valeur angulaire maximale de 15° dans le même plan vertical. Pour revenir à la position de départ, il y a un déplacement latéral de 10° vers l'intérieur. En plus des deux mouvements décrits précédemment, le fémur au cours d'un cycle pivote coaxialement au niveau de la diaphyse d'un angle de ± 6°. L'ensemble des mouvements alternatif et rotatif décrits précédemment détermine pendant un cycle au niveau du genou la figure asymétrique représentée sur la figure 3 dont les limites sont fonction des déplacements angulaires consentis.

Cette disposition assure un balayage total du cotyle par la tête fémorale dans des conditions très proches de celles rencontrées in vivo pour les positions les plus courantes du membre inférieur au niveau du genou (marche avant, recul, changement de direction, etc...).

Pour les essais, la charge 10 appliquée sur l'équipage mobile supportant la prothèse est de 100 daN et on fait tourner le motoréducteur à une vitesse de 25 cycles/min. Au bout de 500 000 cycles, on examine le profil du cotyle et l'on compare la géométrie de celui-ci à celle d'un cotyle en polyéthylène non traité.

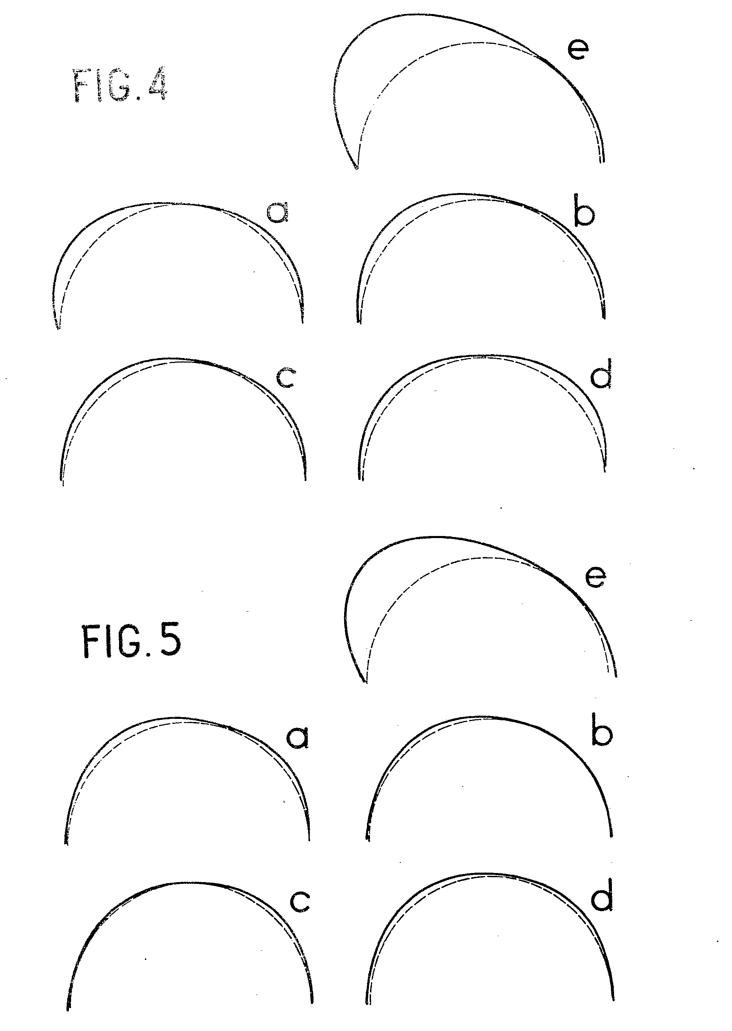

Les résultats obtenus sont illustrés sur la figure 4 qui représente les déformations observées sur le cotyle traité par le procédé de l'invention (4a, 4b, 4c, 4d) et les déformations observées sur un cotyle analogue non traité (4e).

Les figures 4a, 4b, 4c, 4d sont des coupes du cotyle effectuées respectivement selon des plans perpendiculaires à la base de la demi-sphère délimitée par la cavité interne du cotyle et passant par le centre de cette demi-sphère, ces plans étant décalés de 45° les uns par rapport aux autres.

Au vu de cette figure, on constate que le cotyle traité par le procédé de l'invention présente peu de déformations par rapport au cotyle non traité dont la coupe a été effectuée selon le même plan que celui de la figure 4a.

Exemple 2

On utilise une prothèse en polyéthylène identique à celle de l'exemple 1 et on lui applique le même vernis que dans l'exemple 1 sur sa surface externe à l'exception de la cavité interne du cotyle. On soumet la prothèse ainsi revêtue à une irradiation sous vide au moyen d'un faisceau d'électrons d'une énergie de

5

0 076 714

3 MeV, d'une intensité de faisceau de 400 μA, avec une dose de 13 Mrad en orientant la prothèse pour que le faisceau soit perpendiculaire à la concavité de la partie sphérique.

On met ensuite en contact la prothèse irradiée avec du tétrafluoréthylène sous une pression de 1,5 bar à une température de 15 °C pendant 50 heures.

Dans ces conditions, le taux de greffage est de 0,462 mg de tétrafluoréthylène par cm².

On soumet la prothèse ainsi traitée aux tests d'usure réalisés dans les mêmes conditions que dans l'exemple 1. Les résultats obtenus sont donnés sur la figure 5.

Sur cette figure, les références 5a, 5b, 5c et 5d, se rapportent à des coupes réalisées dans les mêmes conditions que les coupes 4a, 4b, 4c et 4d de la figure 4 et la référence 5e se rapporte à une coupe d'un cotyle non traité effectué selon le même plan que la coupe 5a.

Au vu de cette figure, on constate que les déformations du cotyle sont beaucoup moins prononcées que celle du cotyle non traité. De plus, par rapport à la prothèse obtenue dans l'exemple 1, les résultats sont également meilleurs, les déformations étant moins prononcées et plus régulières par rapport au centre de la cavité.

Il semble que dans le cas de l'exemple 1 où la réticulation est effectuée par irradiation avant le greffage, on obtient une réticulation plus élevée du polymère, et de ce fait un taux de greffage de tétrafluoréthylène plus faible car le tétrafluoréthylène qui pénètre par diffusion dans le polymère pénètre moins bien en raison de la réticulation.

Aussi, en raison du taux de réticulation plus élevé et du taux de greffage plus faible, la dureté de la surface de glissement de la prothèse est plus importante et les débris formés sans doute trop abrasifs, ce qui entraîne une usure plus forte de la prothèse.

### Exemple 3

Cet exemple concerne le greffage de la surface de glissement d'une prothèse en polyéthylène identique à celle de l'exemple 1.

On réalise la réticulation et le greffage simultanément en utilisant les conditions expérimentales d'irradiation de l'exemple 2 après avoir protégé la surface externe de la prothèse à l'exception de la cavité interne du cotyle par le même vernis protecteur que dans l'exemple 1.

Après irradiation, on met en contact la prothèse avec du tétrafluoréthylène à la pression de 1,5 bar pendant 65 h à une température de 15 °C puis on enlève le vernis. Dans ces conditions, le taux de greffage obtenu est de 1,32 mg de tétrafluoréthylène par cm². On soumet la prothèse ainsi obtenue à un examen effectué par spectroscopie électronique, ce qui permet de localiser les atomes de fluor.

Ainsi, on constate que ces atomes sont situés à une profondeur allant jusqu'à 3 000 Å, ce qui montre que le greffage est effectué sur une couche de polymère de faible épaisseur.

### Exemple 4

On utilise une prothèse en polyéthylène analogue à celle de l'exemple 1 et on la soumet aux opérations de revêtement et d'irradiation dans les mêmes conditions que celles de l'exemple 2.

Après irradiation, on met en contact la prothèse avec du tétrafluoréthylène à la pression de 1,5 bar, pendant 65 h, à une température de 20 °C. Le taux de greffage obtenu est de 2 mg de tétrafluoréthylène par cm². On examine ensuite la prothèse par spectroscopie électronique pour localiser les atomes de fluor. Dans ce cas, on constate que ces atomes de fluor sont situés sur une profondeur de 4 000 Å.

Ainsi, ce mode de greffage permet de greffer le tétrafluoréthylène sur une faible épaisseur de la surface non protégée de la prothèse.

### Revendications

1. Prothèse articulaire en polymère comportant une surface de glissement, caractérisée en ce que ladite surface de glissement est constituée par une couche d'environ 0,3 à 0,5 μm d'épaisseur en ledit polymère greffé par du tétrafluoréthylène.

2. Prothèse selon la revendication 1, caractérisée en ce que le polymère constituant la prothèse est réticulé.

3. Prothèse selon la revendication 2, caractérisée en ce qu'une partie de ladite prothèse est constituée de polymère réticulé à un degré plus important que le reste de ladite prothèse.

4. Prothèse selon l'une quelconque des revendications 1 à 3, caractérisée en ce que ledit polymère est du polyéthylène.

5. Prothèse selon l'une quelconque des revendications 1 à 3, caractérisée en ce que ledit polymère est choisi dans le groupe comprenant les polyoléfines, le polystyrène, les polyacrylates, le chlorure de polyvinyle, les polyamides et les polyesters.

6. Prothèse selon l'une quelconque des revendications 1 à 5, caractérisée en ce que le taux de greffage en tétrafluoréthylène de la couche d'environ 0,3 à 0,5 μm d'épaisseur constituant la surface de glissement est de 0,3 à 2,5 mg de tétrafluoréthylène par cm² de surface.

7. Procédé de préparation d'une prothèse articulaire selon l'une quelconque des revendications 1 à 6, caractérisé en ce qu'il consiste :

a) à revêtir d'un vernis protecteur, imperméable et inerte vis-à-vis du tétrafluoréthylène, la surface externe d'une prothèse articulaire en polymère comportant une surface de glissement sauf sur la ou les zones qui constituent cette surface de glissement,

b) à soumettre la prothèse ainsi revêtue à une irradiation au moyen de rayonnements ionisants,

c) à mettre en contact la prothèse ainsi irradiée avec de la vapeur de tétrafluoréthylène pendant une durée suffisante pour obtenir le greffage du tétrafluoréthylène sur une épaisseur d'environ 0,3 à 0,5 µm du polymère constituant la surface de glissement, et

d) à éliminer le vernis protecteur.

8. Procédé selon la revendication 7, caractérisé en ce que l'irradiation est réalisée au moyen d'un faisceau d'électrons.

9. Procédé selon la revendication 7, caractérisé en ce que l'irradiation est réalisée en deux étapes, la première étape étant réalisée au moyen de rayonnements gamma et la deuxième étape étant réalisée au moyen d'un faisceau d'électrons.

10. Procédé de préparation d'une prothèse articulaire selon l'une quelconque des revendications 1 à 6, caractérisé en ce qu'il consiste :

a) à irradier une prothèse articulaire en polymère comportant une surface de glissement au moyen de rayonnements ionisants pour réticuler ledit polymère,

b) à revêtir d'un vernis protecteur imperméable et inerte vis-à-vis du tétrafluoréthylène la surface externe de la prothèse articulaire en polymère réticulé à l'exception de la ou des zones qui constituent la surface de glissement,

c) à irradier la prothèse ainsi protégée au moyen de rayonnements ionisants,

d) à mettre en contact la prothèse ainsi irradiée avec de la vapeur de tétrafluoréthylène pendant une durée suffisante pour obtenir le greffage du tétrafluoréthylène sur une épaisseur d'environ 0,3 à 0,5 µm du polymère constituant la surface de glissement et

e) à éliminer le vernis protecteur.

11. Procédé selon la revendication 10, caractérisé en ce que la réticulation est réalisée par irradiation au moyen de rayonnements gamma.

12. Procédé selon l'une quelconque des revendications 10 et 11, caractérisé en ce que l'irradiation de la prothèse protégée par le vernis est réalisée au moyen d'un faisceau d'électrons.

13. Procédé selon l'une quelconque des revendications 7 à 12, caractérisé en ce que l'irradiation est réalisée de façon telle que la prothèse reçoive le rayonnement sous deux orientations différentes afin d'obtenir un degré de réticulation du polymère plus important dans une partie de ladite prothèse.

## Claims

1. Articulated prosthesis of polymeric material having a sliding surface, characterized in that said sliding surface comprises a layer around 0.3 to 0.5 µm in thickness of said polymer grafted with tetrafluoroethylene.

2. Prosthesis according to Claim 1, characterized in that the polymer constituting said prosthesis is cross-linked.

3. Prosthesis according to Claim 2, characterized in that a part of said prosthesis comprises a polymer cross-linked to a greater extent than the remainder of said prosthesis.

4. Prosthesis according to any one of Claims 1 to 3, characterized in that said polymer is polyethylene.

5. Prosthesis according to any one of Claims 1 to 3, characterized in that said polymer is selected from the group comprising polyolefins, polystyrene, polyacrylates, polyvinylchloride, polyamides and polyesters.

6. Prosthesis according to any one of Claims 1 to 5, characterized in that the degree of grafting of tetrafluoroethylene on the layer of around 0.3 to 0.5 µm thickness constituting the sliding surface is 0.3 to 2.5 mg of tetrafluoroethylene per $cm^2$ of surface.

7. Process for preparation of an articulated prosthesis according to any one of Claims 1 to 6, characterized in that it comprises :

a) providing a protective coating, which is impermeable and inert with respect to tetrafluoroethylene, over the external surface of a polymer articulated prosthesis comprising a sliding surface, except over the zone or zones constituting said sliding surface,

b) irradiating the coated prosthesis by means of ionizing radiation,

c) contacting the irradiated prosthesis with tetrafluoroethylene vapour during a period sufficient to provide grafting of tetrafluoroethylene to a thickness of about 0.3 to 0.5 µm of the polymer comprising the sliding surface, and

d) eliminating said protective coating.

8. Process according to Claim 7, characterized in that irradiation is produced by means of an electron beam.

9. Process according to Claim 7, characterized in that irradiation is produced in two stages, the first stage being carried out by means of gamma radiation, and the second stage being carried out by means of an electron beam.

10. Process for production of an articulated prosthesis according to any one of Claims 1 to 6, characterized in that it comprises :

a) irradiating a polymeric articulated prosthesis comprising a sliding surface by ionizing radiation to cross-link said polymer,

b) providing a protective coating, which is impermeable and inert with respect to tetrafluoroethylene, over the external surface of a polymer articulated prosthesis comprising a sliding surface, except over the zone or zones constituting said sliding surface,

c) irradiating the protected prosthesis by means of ionizing radiation,

d) contacting the irradiated prosthesis with tetrafluoroethylene vapour during a period sufficient to provide grafting of tetrafluoroethylene to a thickness of about 0.3 to 0.5 $\mu$m of the polymer comprising the sliding surface, and

e) eliminating said protective coating.

11. Process according to Claim 10, characterized in that cross-linking is produced by irradiation with gamma rays.

12. Process according to either of Claims 10 and 11, characterized in that irradiation of the prosthesis protected by the surface coating is produced by an electron beam.

13. Process according to any one of Claims 7 to 12, characterized in that irradiation is conducted in such a way that the prosthesis receives radiation in two different orientations, whereby to obtain a greater degree of cross-linking of the polymer in one part of said prosthesis.


**Patentansprüche**

1. Gelenkprothese aus einem Polymermaterial mit einer Gleitfläche, dadurch gekennzeichnet, daß die Gleitfläche von einer Schicht von ungefähr 0,3 bis 0,5 $\mu$m Dicke aus dem genannten Polymer besteht, das mit Tetrafluoräthylen veredelt ist.

2. Prothese nach Anspruch 1, dadurch gekennzeichnet, daß das Polymer, das die Prothese bildet, vernetzt ist.

3. Prothese nach Anspruch 2, dadurch gekennzeichnet, daß ein Teil der Prothese aus Polymer besteht, das in einem weitaus höheren Ausmaß vernetzt ist, als der Rest der Prothese.

4. Prothese nach einem der Ansprüche 1 bis 3, dadurch gekennzeichnet, daß das genannte Polymer Polyäthylen ist.

5. Prothese nach einem der Ansprüche 1 bis 3, dadurch gekennzeichnet, daß das Polymer aus der Gruppe gewählt ist, die die Polyolefine, Polystyrol, die Polyacrylate, Polyvinylchlorid, die Polyamide und die Polyester enthält.

6. Prothese nach einem der Ansprüche 1 bis 5, dadurch gekennzeichnet, daß der Tetrafluoräthylen-Veredelungsgrad der Schicht von ungefähr 0,3 bis 0,5 $\mu$m Dicke, die die Gleitfläche bildet, zwischen 0,3 bis 2,5 mg Tetrafluoräthylen pro cm$^2$ der Oberfläche beträgt.

7. Verfahren zur Herstellung einer Gelenkprothese nach einem der Ansprüche 1 bis 6, dadurch gekennzeichnet, daß es umfaßt :

a) Bedecken der Außenfläche einer Gelenkprothese aus Polymer, die eine Gleitfläche aufweist, mit einem Schutzüberzug, der undurchdringlich und inert gegenüber Tetrafluoräthylen ist, mit Ausnahme auf der oder den Bereichen, die die Gleitfläche bilden,

b) Aussetzen der so bedeckten Prothese einer Bestrahlung durch ionisierende Strahlen,

c) Inkontaktbringen der so bestrahlten Prothese mit Tetrafluoräthylen-Dampf für eine Zeitdauer, die ausreichend ist, um eine Veredelung mit Tetrafluoräthylen in einer Schichtdicke von ungefähr 0,3 bis 0,5 $\mu$m des Polymers zu erhalten, das die Gleitfläche bildet, und

d) Entfernen der Schutzschicht.

8. Verfahren nach Anspruch 7, dadurch gekennzeichnet, daß die Bestrahlung mit Hilfe eines Elektronenbündels durchgeführt wird.

9. Verfahren nach Anspruch 7, dadurch gekennzeichnet, daß die Bestrahlung in zwei Stufen durchgeführt wird, von denen die erste Stufe mittels Gammastrahlen und die zweite Stufe mittels eines Elektronenstrahlenbündels ausgeführt wird.

10. Verfahren zum Herstellen einer Gelenkprothese nach einem der Ansprüche 1 bis 6, dadurch gekennzeichnet, daß es umfaßt :

a) Bestrahlen einer Gelenkprothese aus Polymer, die eine Gleitfläche aufweist, mittels ionisierender Strahlen, um das Polymer zu vernetzen,

b) Bedecken der Außenfläche der Gelenkprothese aus vernetztem Polymer mit einer Schutzschicht, die gegenüber Tetrafluoräthylen undurchdringlich und inert ist, mit Ausnahme des oder der Bereiche, die die Gleitfläche bilden,

c) Bestrahlen der so geschützten Prothese mittels ionisierender Strahlen ;

d) Inberührungbringen der so bestrahlten Prothese mit Tetrafluoräthylen-Dampf während einer Zeitdauer, die ausreichend ist, um eine Veredelung mit Tetrafluoräthylen in einer Schichtdicke von ungefähr 0,3 bis 0,5 μm des Polymers zu erhalten, das die Gleitfläche bildet, und

e) Entfernen der Schutzschicht.

11. Verfahren nach Anspruch 10, dadurch gekennzeichnet, daß die Vernetzung durch Bestrahlung mittels Gammastrahlen durchgeführt wird.

12. Verfahren nach einem der Ansprüche 10 und 11, dadurch gekennzeichnet, daß die Bestrahlung der durch den Überzug geschützten Prothese mit Hilfe eines Elektronenstrahlenbündels durchgeführt wird.

13. Verfahren nach einem der Ansprüche 10 bis 12, dadurch gekennzeichnet, daß die Bestrahlung so ausgeführt wird, daß die Prothese die Strahlen unter zwei unterschiedlichen Richtungen empfängt, um einen Vernetzungsgrad des Polymers zu erzielen, der in einem Teil der genannten Prothese viel bedeutender ist.

FIG.1

FIG.2

FIG.3

FIG.4

e

a

b

c

d

FIG.5

e

a

b

c

d